# EUROPEAN PATENT APPLICATION

(11) **EP 4 574 703 A2**
(43) Date of publication of application: **25.06.2025**
(21) Application number: 25168692.9
(22) Date of filing: 01.02.2023
(51) Int. Cl.: B65D 65/40

(54) **ASSEMBLY COMPRISING A SANITARY NAPKIN AND A WRAPPER**

(30) Priority: 16.12.2022 EP 22214316
(62) Divisional of application: 23154539.3
(71) Applicant: Ontex BV, 9255 Buggenhout (BE); Ontex Group NV, 9320 Erembodegem (BE)
(72) Inventor: Descheemaecker, Evan, 9930 Zomergem (BE); Staelens, Eva, 9900 Eeklo (BE); Van Kets, Lies, 9900 Eeklo (BE)
(74) Representative: Saurat, Thibault

(57) **Abstract**

The invention pertains to an assembly (1) comprising a sanitary napkin (10) and a wrapper (30), the sanitary napkin (10) being maintained in a folded configuration by the wrapper (3), the wrapper (3) extends in longitudinal, transversal and vertical directions (L,T,V) and comprises a corresponding length (L), width (W) and thickness (H) respectively, the folded sanitary napkin (10) also extending in said longitudinal, transversal and vertical directions, comprises a corresponding length (L₁₀), width (W₁₀) and thickness (H₁₀), a top surface (4) and a bottom surface (6), the wrapper (30) comprises an upper strip (36) being in contact with the top surface (4) of the folded sanitary napkin (10) and a lower strip (38) being in contact with the bottom surface (6) of the folded sanitary napkin (10), the upper strip (36) and bottom strip (38) each comprising a respective width (W₃₆,W₃₈) and length (L₃₆,L₃₈), the upper and lower strips (36,38) being bonded together at both their longitudinal ends, characterized in that the length (L₃₆,L₃₈) of at least one of the upper or lower strips (36,38) is greater than the length (L₁₀) of the sanitary napkin (10) in a folded state and the width (W₃₆,W₃₈) of both the upper strip and the lower strips (8,10) is lesser than the width (W₁₀) of the sanitary napkin (10) in a folded state.

## Description

### TECHNICAL FIELD

The disclosure pertains to the technical field of absorbent articles for hygiene products and the packaging of such absorbent articles. In particular, the present disclosure relates to individually packaged absorbent articles selected from sanitary napkins, adult incontinence pads or panty liners, which are configured to collect and contain blood, menses, urine, vaginal fluids and avoid leakage. The present disclosure also pertains to the packaging process to wrap or enclose such absorbent articles.

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged in-between said topsheet and backsheet and are typically individually enclosed, or packaged, in a wrapper also commonly called a pouch. These wrappers or pouches are made out of plastic films such as a polypropylene or polyethylene films since these material have several beneficial physical properties such as flexibility, deformability, *etc.* as well as for cost reasons. However, these wrappers are always made from petroleum-derived materials and thus are not environment-friendly.

There is still a need to improve the environmental impact of these wrappers and offer a solution that involves less plastic without degrading the performance of the packaging of these absorbent articles.

The invention thereto aims to provide an environmentally friendly packaging for absorbent articles selected from sanitary napkins, adult incontinence pads or pantyliners.

### SUMMARY OF THE INVENTION

The present invention relates to an assembly comprising a sanitary napkin and a wrapper, the sanitary napkin being maintained in a folded configuration by the wrapper, the wrapper extends in longitudinal, transversal and vertical directions and comprises a corresponding length, width and thickness respectively, the sanitary napkin in a folded state also extends in said longitudinal, transversal and vertical directions, meaning in the same directions as defined by the wrapper, and comprises a corresponding length, width and thickness, the wrapper comprises an upper strip and a lower strip, the upper strip and lower strip each comprising a respective length and width, the upper and lower strips being bonded together at each of, or both, their longitudinal ends characterized in that the length of at least one of the upper or lower strips is greater than the length of the sanitary napkin in a folded state and the width of the upper strip and the width of the lower strips of the wrapper are both lesser than the width of the sanitary napkin in a folded state.

By using a strip that is narrower than the sanitary napkin while maintaining the sanitary napkin in a folded configuration, or folded state, the environmental impact of the packaging is lowered since less material is used.

Preferably, the sanitary napkin comprises in a folded state a top surface and a bottom surface, the upper strip being in contact with a top surface of the folded sanitary napkin and a lower strip being in contact with the bottom surface of the folded sanitary napkin with respect to the vertical direction.

According to an embodiment, the width of the upper strip and the width of the lower strips of the wrapper are both comprised between 5 % to 70 % of the width of the sanitary napkin, preferably between 10 % to 60 % of the width of the sanitary napkin, most preferably between 15 % and 50 % of the width of the sanitary napkin.

According to an embodiment, the length of the upper strip and the length of the lower strips of the wrapper are both greater than the length of the sanitary napkin in a folded state, both strips being bonded together at each of their longitudinal ends in a sealing portion protruding beyond the folded sanitary napkin with respect to the longitudinal direction.

According to an embodiment, the length of one the upper or lower strips is greater than the length of the sanitary napkin in a folded state and the length of the other strip is equal to or lesser than the length of the sanitary napkin, both strips being bonded together at each of their longitudinal end edges in a sealed portion that does not protrudes from the folded sanitary napkin with respect to the longitudinal direction.

According to an embodiment, the upper and lower strips of the wrapper are made of plastic, bioplastic or paper.

According to an embodiment, the upper and lower strips of the wrapper are bonded together at the sealing portions by means of embossing and/or adhesive and/or ultrasonic bonding.

According to an embodiment, the wrapper comprises pre-cuts.

According to an embodiment, the sanitary napkin in a folded state comprises a top surface and a bottom surface, the upper strip being in contact directly and/or indirectly, at least partially, with the top surface of the folded sanitary napkin and the lower strip being in contact directly or indirectly, at least partially, with the bottom surface of the folded sanitary napkin with respect to the vertical direction, an adhesive being arranged at least partially between the top surface and the upper strip and/or between the bottom surface and the lower strip. The adhesive is for example applied in dots in, or along, a portion of the upper and/or lower strips. The upper strip and/or lower strip can be in direct contact with the folded sanitary napkin and/or in contact indirectly with the folded sanitary napkin for example with a layer of adhesive arranged between the folded sanitary napkin and the upper and/or lower strips. The adhesive can be applied along the entire length of the wrapper or along a portion of the length of the wrapper.

The present disclosure also pertains to a container comprising a plurality of assemblies comprising a folded sanitary napkin and a wrapper as described herein.

While these sanitary napkins use less material and thus are environmentally friendly, conventional pouches are also serves as a protection against contaminants and moisture for the sanitary napkin. With such strips, this protection is lowered. While not so necessary to have such protection when staying at home, sanitary napkins being sold in cardboard boxes or plastic bags a user can leave them in their package and grab one when needed, when traveling it can be beneficial to have a reusable container such as a small bag, or a metal box to carry a limited number of sanitary napkins, e.g. one to five napkins. Hence the present disclosure also pertains to a container comprising a plurality of assemblies comprising a folded sanitary napkin and a wrapper according to the present invention. The container is preferably dimensioned to carry a limited number of sanitary napkins such as one, two, three or four napkins.

The present disclosure also pertains to a method suitable for maintaining a sanitary napkin in a folded configuration and forming an assembly as described herein, the method comprising:
- a folding step wherein a sanitary napkin is folded twice or thrice to form a sanitary napkin in a folded configuration;
- a wrapping step wherein an upper strip and a lower strip are provided on each side of the folded sanitary napkin,
- a sealing step wherein said strips are bonded at their longitudinal ends by applying heat and/or pressure and/or ultrasonic vibrations on the strips at the sealing regions;
- a cutting step wherein the strips are cut in the sealing regions.

According to an embodiment, the method further comprises a separation step wherein the individual assemblies are separated from one another, the separation step taking place after the cutting step.

According to an embodiment, in the providing step, the upper and lower strips are applied in the cross direction or the Machine Direction.

According to an embodiment, the method further comprises an additional step of printing a graphic element on said wrapper.

All of these embodiments mentioned above can be taken individually or in combination. Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**Fig. 1** illustrates a schematic cross section of an absorbent article.
**Fig. 2** shows a perspective view of an assembly comprising a sanitary napkin and a wrapper according to an embodiment.
**Fig. 3** depicts a perspective view of an assembly comprising a sanitary napkin and a wrapper according to another embodiment.
**Fig. 4** represents a schematic cross section of an assembly comprising a sanitary napkin and a wrapper according to an embodiment.
**Fig. 5** shows a schematic cross section of an assembly comprising a sanitary napkin and a wrapper according to another embodiment.
**Fig. 6** illustrates a perspective view of a portion of a process suitable for forming an assembly comprising a sanitary napkin and a wrapper.
Fig. 7 depicts a schematic cross section of an assembly comprising a sanitary napkin and a wrapper.

### DETAILED DESCRIPTION OF THE INVENTION

The present invention concerns the packaging of individually packaged absorbent articles selected from sanitary napkins, adult incontinence pads or pantyliners.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles preferably comprise a longitudinal axis defining a length and a transversal axis defining a width, said transversal axis being perpendicular to said longitudinal axis as well as a vertical axis defining a height or thickness. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition distribution layer", "ADL" or "surge management portion" refers to a sub-layer which preferably is a nonwoven wicking layer arranged directly under the topsheet of an absorbent product, which speeds up the transport and improves distribution of fluids throughout the absorbent core. The surge management portion has the ability to quickly collect and temporarily hold liquid surges, and to transport the liquid from its initial entrance point to other parts of the absorbent structure, particularly the retention portion. This configuration can help prevent the exudates from pooling and collecting on the portion of the absorbent garment positioned against the wearer's skin.

The term "adhesive" as used herein is intended to refer to any suitable hot melt, water or solvent borne adhesive that can be applied to a surface of a film layer in the required pattern or network of adhesive areas to form the film-nonwoven laminate of the present disclosure. Accordingly, suitable adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the topsheet to the backsheet and non-structural adhesive for bonding the absorbent article to the wrapper.

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene and polyurethane.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing there through. Examples of breathable backsheet materials are porous polymeric films, nonwoven laminates of spunbond and meltblown layers and laminates of porous polymeric films and nonwoven materials.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

For instance, the basis weight of the layer of cellulosic fibers is preferably measured according to the ISO 536 standard or any standard method. The amount of peeling agent or sealing agent on the wrapper can be measured by taking a sample of the wrapper where the peeling agent or the sealing agent is arranged, weighing said sample before and after removal of the peeling agent or sealing agent (in grams) the difference giving the weight of the peeling agent or sealing agent, and dividing the weight of peeling agent or sealing agent by the area of the sample (in m2) thereby obtaining the amount of peeling agent or sealing agent in gsm.

As used herein, the term "body-facing", "skin-facing" or "bodyside" side or surface means that surface of the article or component which is intended to be disposed toward or placed adjacent to the body of the wearer during ordinary use, while the "outward", "outward-facing" or "garment-facing" side or surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

As used herein, the term "cellulosic" or "cellulosic fibers" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

The term "consisting essentially of' does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

As used herein "configuration", "conformation", "state" are synonymous and interchangeable and means an arrangement of an element, such as the sanitary napkin, in a particular form, figure, or combination.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "garment" means any type of apparel which may be worn. This includes diapers, training pants, incontinence products, surgical gowns, industrial workwear and coveralls, undergarments, pants, shirts, jackets and the like.

The term "graphic" or "graphic element" includes, but is not limited to, any type of design, image, mark, figure, codes, words, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", "bond", "bonded", "affixed", "associated", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

The term "longitudinal", "transversal" and "vertical" as used herein are with respect to the wrapper in an assembled state or in a disassembled or partially assembled state. In other word, the wrapper defines the longitudinal, transversal and vertical directions. The term "in the direction" as used herein means along that direction, e.g. in the longitudinal direction means along the longitudinal direction. With reference to the wrapper, a length corresponds to the dimension extending in the longitudinal direction and is preferably longer than the width, said width being the dimension extending in the transversal direction. The terms "top", "bottom", "upper" and "lower" are all in reference to said vertical direction.

All length, width, and height or thickness can measured with a caliper preferably a micrometer caliper gauge by taking the largest distance separating two points along one direction as defined herein for a given element, e.g. the sanitary napkin in a folded state, the wrapper or the upper strip or the lower strip, or in other words, measuring the distance between two edges of said element along one direction.

The term "length", "width" and "thickness or height" as used herein are respectively in reference to the longitudinal, transversal and vertical direction as defined herein.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layer or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "channels", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5mm to 50 mm, more preferably from 8mm to 40mm, more preferably from 10mm to 30mm, even more preferably from greater than 10mm to less than 25mm.

By "substantially", it is meant at least the majority of the structure referred to.

Use of the term "substrate" includes, but is not limited to, woven or nonwoven webs, porous films, ink permeable films, cellulosic pulp, paper, composite structures, or the like.

Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "pouch", "wrapping sheet", "packaging sheet" or "wrapping construction" are equivalent to "wrapper".

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments maybe combined with one or more other embodiments without departing from the intention of the disclosure and without generalization therefrom.

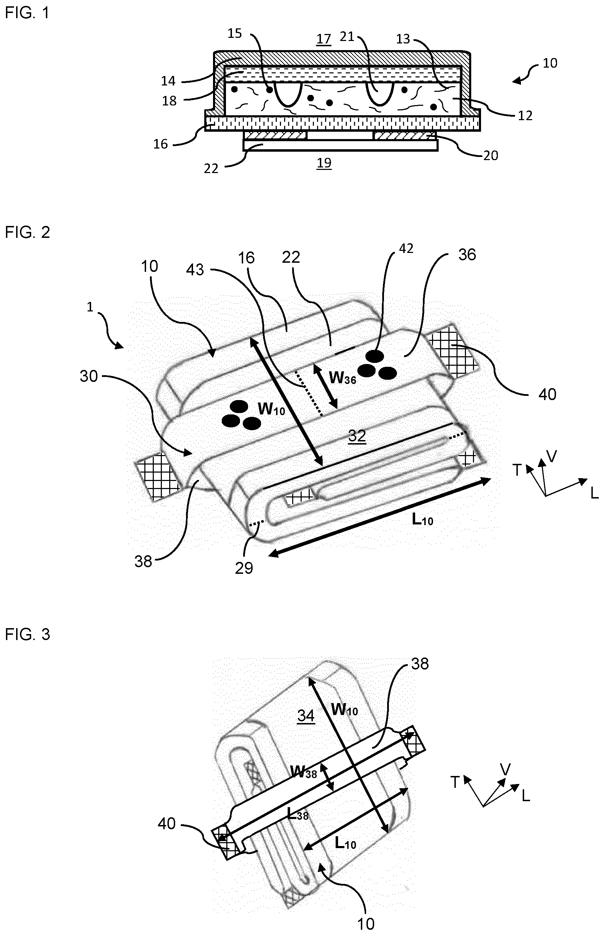

As illustrated in Fig.1, an absorbent article 10, namely a sanitary napkin 10, or a sanitary pad, or a sanitary towel, according to the present disclosure comprises an absorbent core 12 arranged between a liquid permeable topsheet 14 and a liquid impermeable backsheet 16. An optional acquisition distribution layer 18, commonly called ADL, can be positioned between said topsheet 14 and said absorbent core 12. The absorbent core 12 comprises absorbent material selected from the group consisting of cellulose fibers 13 such as fluff pulp, superabsorbent polymers 15 or a combination thereof. Optionally, the absorbent core 12 can comprise at least one channel 21 substantially free of cellulose fibers and/or substantially free of superabsorbent polymer (as illustrated in Fig.1). This at least one channel 21 helps to distribute incoming menstrual fluids throughout the absorbent core 12. The backsheet 16 is joined to the topsheet 14 with the absorbent core 12, and the optional ADL 18, being positioned and enclosed between the topsheet 14 and the backsheet 16. As illustrated in Fig. 1, the topsheet 14 and the backsheet 16 are associated with one another. The topsheet 14 is arranged on the body-facing side 17 or surface whereas the backsheet 16 is arranged on the garment-facing side 19 or surface.

The sanitary napkin 10 comprises on the garment facing side of the backsheet 16 a layer of adhesive 20 so that the sanitary napkin 10 can adhere onto a garment, the layer of adhesive 20 ensuring that the absorbent article 10 stays fastened and secured on the underwear. In order to preserve the integrity of the adhesive 20 before its use, the sanitary napkin 10 comprises a release paper 22 corresponding to a peelable layer destined to protect the adhesive 20.

The release paper 22 is preferably a siliconized paper acting as a release liner. In other words, the release paper 22 comprises a paper layer, comprising cellulosic fibers, and a peeling layer comprising a peeling agent selected from silicon resin series, fluororesin series, octadecilisocyanate series. In other words, the release paper 22 is a paper layer coated with a derivative of silicon, fluororesin or isocyanate. According to an embodiment, the peeling layer comprises a basis weight comprised between 0,3 gsm and 3 gsm, preferably between 0,5 gsm and 1 gsm and the paper layer comprises a basis weight comprised between 10 gsm and 40 gsm. This release paper 22 is recyclable.

The layer of adhesive 20 can be applied on the entire garment facing surface of the backsheet 16, or the layer of adhesive 20 can be applied on a portion of the garment facing surface of the backsheet 16 such as illustrated in Fig. 1 where the layer of adhesive 20 is not as wide or as long as the backsheet 16. The layer of adhesive 20 can be applied in one bloc or in stripes and/or in dots and/or in swirls.

Sanitary napkins 10 are sold in a folded state, either in a three-time fold, meaning comprising two folding lines or that the sanitary napkin has been folded twice, or in a two-time fold, meaning comprising one folding line or that the sanitary napkin has been folded once. Such folding enables to fit more articles in a rectangular carboard box or plastic bag.

As illustrated in FIG. 2, the sanitary napkin 10 is folded in three, meaning comprising two folding lines 29, and is maintained in a folded configuration, or folded state, or folded conformation, by a wrapper 30. In order words, the wrapper 30 acts as clamping mean maintaining the sanitary napkin 10 in a folded state. The present disclosure pertains to an assembly 1 comprising a sanitary napkin 10 and a wrapper 30. The wrapper 30 extends in in the longitudinal, transversal and vertical directions L, T, V as illustrated in FIG. 2, thereby defining a corresponding length L₃₆,L₃₈ in the longitudinal direction, a corresponding width W₃₆,W₃₈ in the transversal direction and a corresponding thickness or height H in the vertical direction. The folded sanitary napkin 10 also extends in said directions, meaning the folded sanitary napkin 10 extends in the same longitudinal, transversal and vertical directions L, T, V as defined by the wrapper 30, thereby defining a corresponding length L₁₀ in the longitudinal direction, a corresponding width W₁₀ in the transversal direction and a corresponding thickness or height H in the vertical direction. Depending on how the sanitary napkin is folded, the length of a folded sanitary napkin 10 can be greater than its width or the width of a folded sanitary napkin 10 can be greater than its length or the length can be equal to the width with respect to the longitudinal, transversal and vertical directions L, T, V as defined by the wrapper 30. For sake of clarity, the length of the wrapper 30 is greater than the width of the wrapper 30.

The sanitary napkin 10 in a folded configuration, i.e. in a folded state, comprises a top surface 32 (FIG. 2) and a bottom surface 34 (FIG.3), both corresponding to the backsheet 16 and/or release paper 22. With respect to the vertical direction, the folded sanitary napkin 10, for example when placed on a flat surface, comprises a top surface 32 and a bottom surface 34 (FIG.3), both corresponding to the backsheet 16 and/or peelable layer 22, the top surface 32 being above the bottom surface 34.

The wrapper 30, defining the longitudinal, transversal and vertical directions L, T, V, comprises an upper strip 36 and a lower strip 38. With respect to the vertical direction V, the upper strip 36 is arranged on top of the assembly 1 and is in contact with the top surface 32 of the sanitary napkin 10 and the lower strip 38 is arranged on the bottom of the assembly 1 and is contact with the bottom surface 34 of the sanitary napkin 10. In other words, the sanitary napkin 10 is partially enclosed, or enveloped, by the wrapper 30. A strip corresponds to a long and narrow piece of paper and/or plastic and/or bioplastic.

The sanitary napkin 10 in a folded state, as illustrated in FIG. 2, comprises a length L₁₀ along the longitudinal direction, a width W₁₀ along the transversal direction T and a thickness along the vertical direction V, said longitudinal, transversal and vertical being defined by the wrapper 30 and the upper and lower strips 36,38. Similarly, the wrapper 30, meaning the upper strip 36 and lower strip 38 each comprise a length L₃₆, L₃₈ along the longitudinal direction, a width W₃₆, W₃₈ along the transversal direction T and a thickness along the vertical direction V.

As illustrated in FIG. 2, the upper and lower strips 36,38 are bonded together at both their longitudinal ends, meaning at each of their longitudinal ends, in a sealing region 40 that are described hereunder.

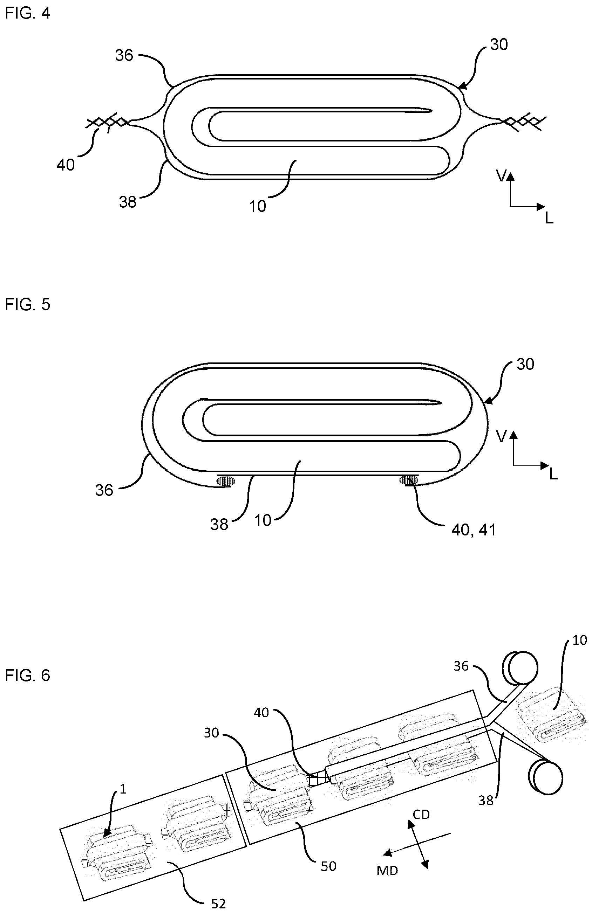

According to the present disclosure, in other to make environmental friendly absorbent article, when the sanitary napkin 10 is in a folded state, the length L₃₆,L₃₈ of at least one of the upper or lower strips 36,38 is greater than the length L₁₀ of the sanitary napkin 10, in a folded state, and the width W₃₆,W₃₈ of both the upper strip and the lower strips 36,38 is lesser than the width W₁₀ of the sanitary napkin 10 in a folded state. In other words, the width W₃₆ of the upper strip 36 and the width W₃₈ of the lower strips 38 of the wrapper 30 are both lesser than the width W₁₀ of the folded sanitary napkin 10 with respect to the transversal direction T as defined herein by the wrapper 30 and the upper and lower strips 36,38 whereas the length L₃₆ of the upper strip 36 and/or the length L₃₈ of the lower strips 38 of the wrapper 30 is/are both greater than the length L₁₀ of the folded sanitary napkin 10 with respect to the longitudinal direction L as defined herein by the wrapper 30 and the upper and lower strips 36,38. FIG. 2 depicts an embodiment where each the upper and lower strips 36,38 comprises a length L₃₆, L₃₈ that is greater than the length L₁₀ of the sanitary napkin 10 in a folded state and FIG. 5 schematically represents an embodiment where the upper strip 36 comprises a length L₃₆ that is greater than the length L₁₀ of the sanitary napkin 10 in a folded state and the lower strip comprises a length L₃₈ that is lesser than the length L₁₀ of the sanitary napkin 10 in a folded state. FIG. 3 illustrates an embodiment where the sanitary napkin 10 has been pivoted, or rotated, by 90° in comparison to the embodiment illustrated in FIG. 2 with the bottom surface 34 of the sanitary napkin and lower strip 38 appearing on top. Here again, in reference to the transversal direction T defined by the wrapper 30 and the upper and lower strips 36,38, the width W₁₀ of the sanitary napkin is greater than the width W_{36,38} of the upper and lower strips 36,38 whereas the length L_{36,38} of the upper and lower strips 36,38 is greater than the length L₁₀ of the sanitary napkin 10 in reference to the transversal direction L defined by the wrapper 30. In other words, the width W₃₆,W₃₈ of the upper and lower strips 36,38 are both inferior to the width W₁₀ of the sanitary napkin 10 in a folded state.

According to an embodiment, the width W₃₆,W₃₈ of both the upper and lower strips 36,38 of the wrapper 30 are comprised between 5 % to 70 % of the total width W₁₀ of the sanitary napkin 10 in a folded state, preferably from 10 % to 60 % of the total width W₁₀ of the sanitary napkin 10, preferably between 15 % and 50 % of the total width W₁₀ of the sanitary napkin 10.

According to an embodiment as illustrated in FIG. 2 to 4, both length L₃₆,L₃₈ of the upper and lower strips 36,38 is greater than the length L₁₀ of the sanitary napkin 10 in a folded state. Both strips 36,38 are bonded together at their respective longitudinal ends in a sealing region 40 that is protruding from the folded sanitary napkin 10 with respect to the longitudinal direction L.

According to an embodiment as schematically illustrated in FIG. 5, the length L₃₆,L₃₈ of one the upper or lower strip 36,38 is greater than the length L₁₀ of the sanitary napkin 10 in a folded state. The length L₃₆,L₃₈ of the other strip 36,38 is equal to or lesser than the length L₁₀ of the sanitary napkin 10 in a folded state. Here the length of the lower strip 38 is lesser than the length of the sanitary napkin 10 in a folded state. Both strips 36,38 are bonded together at their respective longitudinal ends in a sealing portion 40 that does not protrude from the folded sanitary napkin 10 with respect to the longitudinal direction L. In other words, the sealing portion 40 is arranged under or above the sanitary napkin 10 with respect to the vertical direction.

Hence the wrapper 30 and upper and lower strips 36,38 according to the present disclosure still acts as a clamping mean, or a holding mean, for the sanitary napkin 10 and keeps it in a folded configuration while saving on material and thus being more environmentally friendly. Additionally, by not using a conventional pouch, there is no need to add a closing tape with the present assembly 1 again saving on material.

The upper and lower strips 36, 38 of the wrapper 30 are made of a plastic material such as polyethylene and/or bioplastic and/or paper. While bioplastic and paper may be more sustainable material, using a plastic material such as polyethylene can also simplify the process namely for the sealing step as we will see hereunder. According to an embodiment the wrapper 30, for example the upper and lower strips 36,38 both comprise a layer consisting essentially of cellulosic fibers, meaning paper, with a basis weight comprised between 10 gsm and 120 gsm. According to another embodiment, the wrapper 30 for example the upper and lower strips 36,38 both comprise a paper layer laminated with a polyethylene film. According to another embodiment, the wrapper 30, for example the upper strip 36 comprises a layer of paper and the lower strip 38 comprises a layer consisting essentially of bioplastic. In other words, the upper and lower strip 36,38 can be made out of a same material or be made of different materials or comprise a laminate of two or more materials.

Plastic, bioplastic and paper are materials that can easily be printed on, the wrapper 30 according to the present disclosure can be provided with graphic elements that are printed, by common techniques such as flexographic printing, gravure printing or inkjet printing, onto the outer surface and/or inner surface 23 of the wrapper 30, in particular onto the top surface of the upper strip 36 and/or the bottom surface of the lower strip 38 with respect to the vertical direction V.

According to a further embodiment, the release paper 22 and/or the backsheet 16 can also comprise a graphic element printed upon its surface. The graphic element on the wrapper, meaning on the upper strip 36 and/or on the lower strip 38, can be matching or congruent. By congruent it is meant capable of being placed over another figure and exactly matching, for example if a circle is printed on the release paper 22, the wrapper 30 comprises two arcuate lines matching the partial periphery of said circle. The graphic element printed on the release paper 22 and/or backsheet 16 can be of the same nature as the graphic element printed on the wrapper 30 or of different nature. For example the release paper 22 and/or backsheet 16 can comprise a pattern printed upon its outer surface whereas the wrapper comprises one or more words printed upon its outer surface or the example the release paper 22 and/or backsheet 16 and the wrapper can all comprise a drawing printed upon its surface. According to a further embodiment, the release paper 22, the backsheet 16 and the wrapper 30 each comprises a graphic element.

As illustrated in FIG. 2 and 3, the absorbent article 10 once folded and wrapped is kept in a folded state by the wrapper 30 as described herein, said wrapper 30 is arranged to partially cover the release paper 22. In other words, the wrapper 30 is arranged to cover only a portion, or a part, of the release paper 22, meaning not the entirety of the release paper 22. Similarly, the wrapper 30 is arranged to cover only a portion, or a part, of the backsheet 16, meaning not the entirety of the backsheet 16. This enables to save on raw material.

According to an embodiment, the wrapper 30 as described herein is dimensioned to cover the entirety of the release paper 22, meaning the width of the upper strip W36 and/or lower strip W38 is equal to the width of the release paper 22 and the wrapper 30 covers only a portion or the backsheet 16. "Equal" as used herein encompasses substantially equal meaning that the width of the upper strip 36 and/or lower strip 38 can be a little bit greater or lesser than the width of the release paper 22, for example up to 10 % greater or lesser, e.g. the width of the upper strip 36 is 5% greater than the width of the release paper 22. This enables to save on raw material.

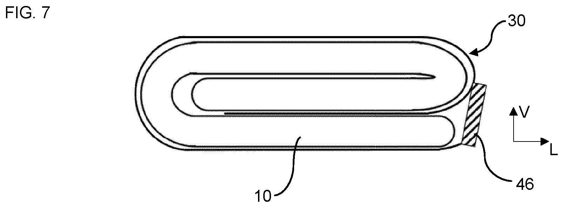

According to a further embodiment, the release paper 22 is removed, meaning the release paper 22 is absent from the assembly 1, also meaning that the wrapper 30 as described herein is directly covering the adhesive layer 20. In this further embodiment, the wrapper 30 has the same width, it can be larger, as the release paper 22 so that it protects the layer of adhesive 20 to be applied on the garment. The wrapper 30 preferably comprises heat-sealable material at its longitudinal ends to enable a sealing of the wrapper 30 in sealing portions 40, or sealing regions 40, and a peeling material such as siliconized paper in between the sealing portions 40 to enable an easy removal of the wrapper 30 without damaging the layer of adhesive 20 and ensure a better securing of the sanitary napkin 10 to the garment. Sealing regions 40 and sealing portions 40 are interchangeable terms. Alternatively, as illustrated in FIG. 7, the wrapper 30 comprises one, two or more strips that have a width that is lesser than the width of the backsheet 16, the wrapper 30 comprises only peeling material, e.g. siliconized paper, and a closing mean 46, such as a closing tape, linking one extremity of the wrapper 30 to a surface of the wrapper 30 that is arranged on the longitudinal half of the wrapper 30 that is opposite to said one extremity. The wrapper 30 maintains the sanitary napkin 10 in a folded state. In the specific embodiment where the wrapper comprises only one strip that is directly covering the adhesive layer 20, said unique strip acts as both the upper and lower strips as described herein. This single strip defines the longitudinal, transversal and vertical directions, the unique strip is longer than the sanitary napkin 10, namely the backsheet 16, in a folded configuration with respect to the longitudinal direction and is shorter than the sanitary napkin 10, namely the backsheet 16, with respect to the transversal direction. In other words, in this embodiment, the wrapper 30 comprises two halves with respect to the longitudinal direction L forming a continuity of matter, meaning the wrapper 30 comprises a monolithic structure. One half corresponds to the upper strip 36 and covers the top surface of the sanitary napkin 10 and the other half corresponds to the lower strip 38 and covers the bottom surface of the sanitary napkin with respect to the vertical direction. Each half are equal in length and width. The two halves are bonded together by the closing tape 46. As described herein the length of the unique strip is greater than the length of the sanitary napkin 10 in a folded state and the width of the unique strip is lesser than the width of the sanitary napkin 10 in a folded state.

As discussed above, the upper strip 36 and the lower strip 38 are joined together at both their longitudinal ends each in a sealing portion 40. In other words, a longitudinal end of the upper strip 36 is joined to a longitudinal end of the bottom strip 38 and the other longitudinal end, i.e. the end arranged at the opposite end with respect to the longitudinal direction L, of the upper strip 36 is joined to the other longitudinal end of the bottom strip 38. In other terms, the upper strip 36 and the lower strip 38 are superposed, meaning arranged in a facing relationship, and are affixed directly to each other at their longitudinal ends.

The upper and lower strips 36, 38 of the wrapper 30 are bonded together at sealing portions 40 by means of thermo-sealing and/or pressure embossing and/or adhesive and/or ultrasonic bonding. Thermo-sealing is carried out by applying heat to a heat-sealable material. Pressure embossing is carried out by embossing rollers which apply pressure on the wrapper 30, i.e. on the upper and lower strips 36, 38, the compression leading to an entanglement of the fibers. The pressure embossing, or mechanical embossing, can be carried without adhesive preferably by apply a stronger pressure to promote the intertwining, or entanglement, of fibers as schematically illustrated in FIG. 4. The pressure embossing can also be applied with an adhesive 41 to better secure the strips together. An adhesive 41 can also be applied on one or both of the strips 36,38, namely on the side of the strip 36,38 that is facing the other strip 36,38. For example in the embodiment as illustrated in FIG. 2, the adhesive is applied on the bottom side of the upper strip 36 and on the top side of the lower strip 38. For example in the embodiment as illustrated in FIG. 5 the adhesive 41 is applied on the top side of the longitudinal end of the upper strip 36 and on the bottom side of the lower strip 38 with respect to the vertical direction.

For the embodiments implying the use of an adhesive 41 and/or a heat-sealable material 41, it is preferable that the adhesive 41 and heat-sealable material 41 comprise a non-petroleum derived material as to lower the environmental impact of the absorbent article. The adhesive and/or heat-sealable material are preferably selected from and not limited to polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), a polymer material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The adhesive and/or heat-sealable material preferably comes from a sustainable source, or bio-based or bioplastic, and is biodegradable and/or compostable. Preferably, the adhesive 41 used for joining the upper and lower strips 36,38 is the same as the adhesive 20 used for bonding the backsheet 26 and the release paper 22.

The wrapper 30, e.g. the upper strip 36 and/or the lower strip 38, comprise pre-cuts 43 so that a user can tear the wrapper 30 more easily before unfolding and applying the sanitary napkin 10 in a garment. In other words, the wrapper 30 comprises a weakened division line 43 so that a user can induce a rip along the weakened division line 43 in a tearing motion. Weakened division lines 43 can be applied by cutting, perforating, stretching, scratching such as with the use of a die cutter or a knife cutter, and the like. Weakened division lines 43 can also be applied by chemical treatment such as the application of acid or thermal treatment such as a heating, partial burning or freezing.

According to an embodiment, the sanitary napkin 10 in a folded state comprises a top surface 32 and a bottom surface 34, the upper strip 36 being in contact with the top surface 34 of the folded sanitary napkin 10 and the lower strip 38 being in contact with the bottom surface 34 of the folded sanitary napkin 10 with respect to the vertical direction, an adhesive 42 being arranged at least partially between the top surface 32 and the upper strip 36 and/or between the bottom surface 34 and the lower strip 38. The adhesive 42 is for example applied in dots in, or along, a portion, with respect to the longitudinal direction, of the upper and/or lower strips 36,38. The upper strip 36 and/or lower strip 38 can be in direct contact with the folded sanitary napkin 10 and/or in contact indirectly with the folded sanitary napkin 10 for example with a layer of adhesive 42 being arranged between the folded sanitary napkin 10 and the upper and/or lower strips 36,38. The adhesive 42 can be applied along the entire length of the wrapper or along a portion of the length of the wrapper. The adhesive 42 can be the same as the adhesive 20 for the release paper 22 or the same as the adhesive 41 used for the sealing portion 40. The adhesive 42 enables to maintain or hold the upper and/or lower strip 36,38 onto the folded sanitary napkin 10 and avoid that said strips slip off.

It is possible to combine the features of the different embodiments mentioned hereabove. For example a wrapper 30 can comprise a graphic element congruent or matching the graphic element of the release paper 22 and pre-cuts 43 i.e. a weakened division line 43. For example, a wrapper 30 can comprise a closing tape 46 and a graphic element. The wrapper 30 can be arranged, or applied, in the cross direction and comprise pre-cuts 43 and/or a graphic element.

The present disclosure also pertains to a method suitable for maintaining a sanitary napkin 10 in a folded configuration and forming an assembly 1 as described herein. The method is partially illustrated in FIG. 6, the method comprising:
- a folding step wherein a sanitary napkin 10 is folded twice or thrice to form a sanitary napkin 10 in a folded configuration;
- a wrapping step wherein an upper strip 36 and a lower strip 38 are provided on each side of the folded sanitary napkin 10, meaning on the top surface 32 and the bottom surface 34 of the sanitary napkin 10,
- a sealing step wherein said strips 36,38 are bonded at their longitudinal ends by applying heat and/or pressure and/or ultrasonic vibrations on the strips 36,38 at the sealing regions 40;
- a cutting step wherein the strips 36,38 are cut in the sealing regions 40.

The method can further comprises a separation step, where the individual assemblies 1 are separated from one another, the separation step taking place after the cutting step.

The method can further comprises one or more gluing steps, where the adhesives 21,42 are applied on the lower strip 36 and/or upper strip 38.

The method further comprises a printing step where a graphic element is printed on said wrapper 30. Alternatively, the method can comprise a wrapping step as described hereabove where the upper and/or lower strips 36,38 are pre-printed, meaning already printed with a graphic element.

The sealing step can be carried out at a bonding station, the bonding station comprising for example two embossment rollers, an applicator of adhesive eventually combined with two rollers, a sonotrode with an anvil or an embossment roller.

The cutting step can be carried out at a cutting station, the cutting station comprising for example two rollers, one roller comprising a knife and the other roller acting as an anvil.

The printing step can be carried out at a printing station for example using flexography or ink jet printing as a printing method.

The separation step can be carried out at a separation station, the separation station comprising for example a first and second conveying belts 50,52. The second conveying belt 52 is arranged downstream, with respect to the machine direction, or conveyance flow, of the assemblies 1, of the first conveying belt 50 and goes at a faster speed than the first conveying belt 50 so to induce a gap between two successive assemblies 1 as described herein.

According to an embodiment, the upper and lower strips 36,38 are applied in the cross-direction CD to form an assembly 1 as illustrated in FIG. 3 or in the Machine Direction MD to form an assembly 1 as illustrated in FIG. 2.

More specifically, the ultrasonic bonding is executed by an ultrasonic welding device, such as a sonotrode, inducing vibrations at a frequency between 10 kHz and 50 kHz, apply an energy on the wrapper. The ultrasonic welding device can be associated with an embossing roller. Material such as plastics or bioplastics can melt at lower temperatures than paper however it is possible to use two strips made out of paper and add a sealing agent in between that will melt thereby binding the two strips. The ultrasonic welding device comprises a vibration generator, for example and not limited to, a magnetostrictive or piezoelectric transducer, said generator is attached to a tapering rod or probe commonly called a sonotrode usually made out of metal, such as and not limited to titanium, aluminum or steel. The sonotrode is submitted to the vibrations generated by the generator and said sonotrode then applies this vibrational energy to the combined upper and lower strips 36,38. The vibrational energy causes the at least partial melting of the upper or lower strip 36,38 and/or the adhesive 41 so as to join the upper and lower strips 36,38 in these melting points which can be considered as the sealing regions 40 or bonding regions.

It is supposed that the present invention is not restricted to any form of realization described previously and that some modifications can be added to the presented example of fabrication without reappraisal of the appended claims.

## Claims

1. Assembly (1) comprising a sanitary napkin (10) and a wrapper (30), the sanitary napkin (10) being maintained in a folded configuration by the wrapper (30), the wrapper (30) extends in longitudinal, transversal and vertical directions (L,T,V) and comprises a corresponding length, width and thickness respectively, the sanitary napkin (10) in a folded state also extends in said longitudinal, transversal and vertical directions and comprises a corresponding length (L₁₀), width (W₁₀) and thickness (H₁₀), the wrapper (30) comprises a unique strip (36,38) comprising a respective length (L₃₆,L₃₈) and width (W₃₆,W₃₈), **characterized in that** the length (L₃₆,L₃₈) of the unique strip (36,38) is greater than the length (L₁₀) of the sanitary napkin (10) in a folded state and the width (W₃₆,W₃₈) of the unique strip (36,38) is lesser than the width (W₁₀) of the sanitary napkin (10) in a folded state.

2. Assembly (1) comprising a folded sanitary napkin (2) and a wrapper (3) according to claim 1, wherein the width (W₃₆,W₃₈) of the unique strip (36,38) of the wrapper (30) is comprised between 5 % to 70 % of the width (W₁₀) of the sanitary napkin (10), preferably between 10 % to 60 % of the width (W₁₀) of the sanitary napkin (10), most preferably between 15 % and 50 % of the width (W₁₀) of the sanitary napkin (2).

3. Assembly (1) comprising a folded sanitary napkin (10) and a wrapper (30) according to claim 1 or 2, wherein the length (L₃₆,L₃₈) of the unique strip (36,38) of the wrapper (30) is greater than the length (L₁₀) of the sanitary napkin (10) in a folded state, both strips (36,38) being bonded together at each of their longitudinal ends in a sealing portion (40) protruding beyond the folded sanitary napkin (2) with respect to the longitudinal direction (L).

4. Assembly (1) comprising a folded sanitary napkin (10) and a wrapper (30) according to any of the preceding claims, **characterized in that** the unique strip (36,38) of the wrapper (30) is made of plastic, bioplastic or paper.

5. Assembly (1) comprising a folded sanitary napkin (10) and a wrapper (30) according to any of the preceding claims, **characterized in that** the unique strip (36,38) of the wrapper (30) comprises two halves, said halves being bonded together by a closing tape (46).

6. Assembly (1) comprising a folded sanitary napkin (10) and a wrapper (30) according to any of the preceding claims, **characterized in that** the wrapper (30) comprises pre-cuts (43).

7. Assembly (1) comprising a folded sanitary napkin (10) and a wrapper (30) according to any of the preceding claims, **characterized in that** the sanitary napkin (10) in a folded state comprises a top surface (32) and a bottom surface (34), the upper strip (36) being in contact with the top surface (34) of the folded sanitary napkin (10) and the lower strip (38) being in contact with the bottom surface (34) of the folded sanitary napkin (10) with respect to the vertical direction, an adhesive (42) being arranged at least partially between the top surface (32) and the upper strip (36) and/or between the bottom surface (34) and the lower strip (38).

8. A container comprising a plurality of assemblies (1) comprising a folded sanitary napkin (10) and a wrapper (30) according to any of the preceding claims.
